(19) Europäisches Patentamt
European Patent Office
Office européen des brevets

(11) EP 3 935 939 A1

(12) **EUROPEAN PATENT APPLICATION**
published in accordance with Art. 153(4) EPC

(43) Date of publication:
**12.01.2022 Bulletin 2022/02**

(21) Application number: **20770725.8**

(22) Date of filing: **06.03.2020**

(51) Int Cl.:
***A01H 1/00*** *(2006.01)* ***C12N 5/10*** *(2006.01)*
***C12N 15/82*** *(2006.01)* ***C12Q 1/6869*** *(2018.01)*

(86) International application number:
**PCT/JP2020/009553**

(87) International publication number:
**WO 2020/184402 (17.09.2020 Gazette 2020/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2019 JP 2019042535**

(71) Applicant: **National University Corporation Kobe University**
**Kobe-shi, Hyogo 657-8501 (JP)**

(72) Inventors:
- **KATAYAMA, Kenta**
**Kobe-shi, Hyogo 657-8501 (JP)**
- **NISHIDA, Keiji**
**Kobe-shi, Hyogo 657-8501 (JP)**

(74) Representative: **Heubeck, Christian**
**Weickmann & Weickmann**
**Patent- und Rechtsanwälte PartmbB**
**Postfach 860 820**
**81635 München (DE)**

# (54) METHOD FOR SELECTING PROMOTER THAT FUNCTIONS IN ORGANELLE

(57) The present invention provides a method for selecting a promoter that functions in an organelle, the method comprising: (1) the step of preparing sequence information obtained by RNA sequencing analysis; (2) the step of mapping the sequence information prepared in the step (1) onto a sequence of organellar DNA; (3) the step of calculating the amount of change in RNA expression in each region based on the mapping information obtained in the step (2); (4) the step of selecting regions in which the amount of change obtained in the step (3) is within a range of preset reference values; and (5) the step of identifying a region, among the regions selected, as a promoter functioning in an organelle.

EP 3 935 939 A1

SEQUENCE LISTING

<110> NATIONAL UNIVERSITY CORPORATION KOBE UNIVERSITY

<120> Method for screening promoter functioning in an organelle

<130> 092996

<150> JP 2019-042535
<151> 2019-03-08

<160> 37

<170> PatentIn version 3.5

<210> 1
<211> 229
<212> DNA
<213> Arabidopsis thaliana

<400> 1
```
ggcctttacg ttttcaaatg gaatcgataa gatcgttcta gtcgacaata ttaaaattct      60

aattttgaaa gcgggggac ggaagttaca tatacaaata caagaacttc ttaattacat      120

gtacatctgt aattatatat attactatat ataatgtaat acaataaaga agaaagaagg      180

aggatttcta atgcgagatc taaaaacata tctttccgta gcaccggta                 229
```

<210> 2
<211> 108
<212> DNA
<213> Arabidopsis thaliana

<400> 2
```
tccattttac attggttgac atggctatat aagtcatgtt atactgtttc ataacaagct      60

ctcaattatc tacttagaga atttgtgcgc ttgggagtcc ctgattat                  108
```

<210> 3
<211> 368
<212> DNA
<213> Arabidopsis thaliana

<400> 3
```
catggatgaa ttccgcatat tgtcatatct aggatttaca tatacaacag atattactgt      60

caagagtgat tttattaata ttttaatttt aatattaaat atttggattt ataaaaagtc      120

aaagattcaa aacttgaaaa agaagtatta ggttgcgcta tacatatgaa agaatataca      180

ataatgatgt atttggcgaa tcaaatatca tggtctaata aagaataatt ctgattagtt      240

gataattttg tgaaagattc ctgtgaaaaa ggttaattaa atctattcct aatttatgtc      300

gagtagacct tgttgttttg ttttattgca agaattctaa attcatgact tgtagggagg      360

gacttatg                                                              368
```

<210> 4

<211> 167
<212> DNA
<213> Arabidopsis thaliana

<400> 4
ctgcagtttt gggctttggc ggtatttatc atgcacttct gggacccgaa actcttgaag 60

aatcttttcc ctttttcggt tatgtatgga aagatagaaa taaaatgacc accattttgg 120

gtattcactt aattttgtta ggtgtaggtg cctttcttct agtattc 167

<210> 5
<211> 104
<212> DNA
<213> Arabidopsis thaliana

<400> 5
ggctggatta atcttagcga ttacttaatt agaattacgt cctaagtcat tggatgattg 60

tatcattaac tatttcttta ttttggtgtg aggaacttat catg 104

<210> 6
<211> 239
<212> DNA
<213> Arabidopsis thaliana

<400> 6
gacacggtta tacatcgaca agcaagaaaa aaagaaatta tgtaacaccc catttctata 60

ctaattcaaa ttgcgttgct gtgtcagaag aaggatagct atactgattc ggtagactct 120

aaaaataccc ttggtacttt attgacgatc tcacaaagat gcaatctcag tgaattttaa 180

tttacttatt catcttttac agaatcgatt ccctacgaga atatctggag ttcagtatg 239

<210> 7
<211> 189
<212> DNA
<213> Arabidopsis thaliana

<400> 7
ccgttgagca ccctatggat atgtcataat agatccgaac acttgcccca gatcgacttc 60

cagatcataa ttgctctagt gaataactaa agaaaataga tgaatagatg gaagatagaa 120

gagaaagaaa aaaaaattct aagtatctat catcggttca ctaattactt gagtgactgt 180

tggcgggtt 189

<210> 8
<211> 207
<212> DNA
<213> Arabidopsis thaliana

<400> 8
ttgcttttca aagatttatg aaggattata ttaattcgaa aacatttcta tttaggtttg 60

aaatcgcgtg ctttttttatt gtccccttttg aaaaaaaaat atatatatac aaaccagtga 120

ataagaaaaa ataaaattaa gaataaaata aaaaggattt tttattttat ggaacataca 180

tatcaatatt catggatcat ccctttc 207

<210> 9
<211> 247
<212> DNA
<213> Arabidopsis thaliana

<400> 9
ctttatctga ataatgagtc atccgacaat tcatgattta gattcaacta cttatactta 60

ttaataaact aatagcaagg aagaaacaaa ttgagttgat ccgtttacct aagtaaggac 120

caataaaatc aaaaattttg atcttcgaaa ccaattaaat gaaattctaa gggttccatt 180

ttatggggca gtgcgcgaga aattaaatca taaacaaatg atagaatttg agcgccctga 240

aaatgcc 247

<210> 10
<211> 122
<212> DNA
<213> Arabidopsis thaliana

<400> 10
gtgtgatttg tttagttggg atccaaaact aaaatataaa atttaagtaa ataagtaaaa 60

aaaaaggggg ggtcttgaat caaaataatt taaagttctt atttctgtca gagggcaata 120

tg 122

<210> 11
<211> 336
<212> DNA
<213> Arabidopsis thaliana

<400> 11
aaacatgtgg gcgcaaaatg ctatcctgct ctattgttac gtagcaatag aagcccgctc 60

atgctgcttc ggcggcgctt tttcgccttc tcttcgttct gggcaggagc gagaagccac 120

tcgactaaaa ggtactgacg taactctcaa ctaaaaaaag gagaggaacc ctaatgactc 180

gactaaaagg agaggaaccc taatgactcg actaaaagga gaggaaccct aatgactcga 240

ctaaaaggag aggaacccta gtgactcgac tgaaaaggag aggataggag cgctagtgga 300

cacggggagg gaaagaagac tggaaccgtg catctt 336

<210> 12
<211> 163
<212> DNA
<213> Arabidopsis thaliana

<400> 12
tctagttagt agactcagaa aggcattgta tggactaaaa caaaactccg gctcgcaatt 60

cttcctaagc gggaagaccc gtggatatgg tcttcaaccc atacccatta ggagtaccct 120

cacgaatgaa tgtaggacag atatttgaat gctcgcttgg gtt 163

<210> 13
<211> 21
<212> DNA
<213> Artificial Sequence

<220>
<223> PCR Primer

<400> 13
gtgtgatttg tttagttggg a 21

<210> 14
<211> 28
<212> DNA
<213> Artificial Sequence

<220>
<223> PCR Primer

<400> 14
catattgccc tctgacagaa ataagaac 28

<210> 15
<211> 28
<212> DNA
<213> Artificial Sequence

<220>
<223> PCR Primer

<400> 15
ttgcttttca aagatttatg aaggatta 28

<210> 16
<211> 30
<212> DNA
<213> Artificial Sequence

<220>
<223> PCR Primer

<400> 16
gaaagggatg atccatgaat attgatatgt 30

<210> 17
<211> 25
<212> DNA
<213> Artificial Sequence

<220>
<223> PCR Primer

<400> 17
ccgttgagca ccctatggat atgtc 25

<210> 18
<211> 20
<212> DNA
<213> Artificial Sequence

<220>
<223> PCR Primer

<400> 18
aacccgccaa cagtcactca 20

<210> 19
<211> 26
<212> DNA
<213> Artificial Sequence

<220>
<223> PCR Primer

<400> 19
ctttatctga ataatgagtc atccga 26

<210> 20
<211> 20
<212> DNA
<213> Artificial Sequence

<220>
<223> PCR Primer

<400> 20
ggcatttca gggcgctcaa 20

<210> 21
<211> 26
<212> DNA
<213> Artificial Sequence

<220>
<223> PCR Primer

<400> 21
gacacggtta tacatcgaca agcaag 26

<210> 22
<211> 34
<212> DNA
<213> Artificial Sequence

<220>
<223> PCR Primer

<400> 22
catactgaac tccagatatt ctcgtaggga atcg 34

<210> 23
<211> 20

<212> DNA
<213> Artificial Sequence

<220>
<223> PCR Primer

<400> 23
ggctggatta atcttagcga 20

<210> 24
<211> 21
<212> DNA
<213> Artificial Sequence

<220>
<223> PCR Primer

<400> 24
catgataagt tcctcacacc a 21

<210> 25
<211> 20
<212> DNA
<213> Artificial Sequence

<220>
<223> PCR Primer

<400> 25
ctgcagtttt gggctttggc 20

<210> 26
<211> 27
<212> DNA
<213> Artificial Sequence

<220>
<223> PCR Primer

<400> 26
gaatactaga agaaaggcac ctacacc 27

<210> 27
<211> 26
<212> DNA
<213> Artificial Sequence

<220>
<223> PCR Primer

<400> 27
ggcctttacg ttttcaaatg gaatcg 26

<210> 28
<211> 19
<212> DNA
<213> Artificial Sequence

<220>
<223> PCR Primer

<400> 28
taccggtgct acggaaaga 19

<210> 29
<211> 26
<212> DNA
<213> Artificial Sequence

<220>
<223> PCR Primer

<400> 29
tccattttac attggttgac atggct 26

<210> 30
<211> 23
<212> DNA
<213> Artificial Sequence

<220>
<223> PCR Primer

<400> 30
ataatcaggg actcccaagc gca 23

<210> 31
<211> 30
<212> DNA
<213> Artificial Sequence

<220>
<223> PCR Primer

<400> 31
catggatgaa ttccgcatat tgtcatatct 30

<210> 32
<211> 23
<212> DNA
<213> Artificial Sequence

<220>
<223> PCR Primer

<400> 32
cataagtccc tccctacaag tca 23

<210> 33
<211> 18
<212> DNA
<213> Artificial Sequence

<220>
<223> PCR Primer

```
<400>  33
aaacatgtgg gcgcaaaa                                                    18


<210>  34
<211>  19
<212>  DNA
<213>  Artificial Sequence

<220>
<223>  PCR Primer

<400>  34
aagatgcacg gttccagtc                                                   19


<210>  35
<211>  31
<212>  DNA
<213>  Artificial Sequence

<220>
<223>  PCR Primer

<400>  35
tctagttagt agactcagaa aggcattgta t                                     31


<210>  36
<211>  25
<212>  DNA
<213>  Artificial Sequence

<220>
<223>  PCR Primer

<400>  36
aacccaagcg agcattcaaa tatct                                            25


<210>  37
<211>  12
<212>  PRT
<213>  Artificial Sequence

<220>
<223>  mitochondrial localization signal

<400>  37

Met Leu Ser Leu Arg Gln Ser Ile Arg Phe Phe Lys
1               5                   10
```

## Description

[Technical Field]

The present invention relates to a method for selecting a promoter that functions in an organelle, and a promoter DNA comprising a novel sequence selected by the method.

[Background Art]

Improving plants with useful traits that cannot be added by cross breeding by introducing foreign genes into plant cells is extremely significant for future improvement of crops and development of agriculture, such as contributing to the promotion of increased food production and environmental improvement, amid the rapidly changing agricultural situations including rapid population growth and climate change in recent years. In addition, from the perspective of restricting the supply of fossil fuels, etc., technical improvement is indispensable for gene recombination techniques for plants in order to achieve the production of substances by plants using carbon dioxide as a raw material.

As one of the gene recombination techniques for plant cells, a gene recombination technique is known that targets organellar DNA, such as plastids (e.g., chloroplasts) and mitochondria, instead of the nucleus (for example, Non Patent Literature 1). The organelles are usually tens to thousands in cells (for example, in the case of chloroplasts, there are about 100 organelles per cell), and there are usually dozens to hundreds of genomes per organelle, where high expression of the genes introduced into the organelle is thus expected. Furthermore, even proteins or ribonucleic acids that are harmful in the cytoplasm can be accumulated in organelles without specific cleavage or modification in each organism, and there is no silencing, so that gene expression is stable even in progeny. Moreover, the organelles are often limited to maternal or paternal inheritance, and thus, the risk of spreading foreign genes is also extremely low. In order to express proteins stably and efficiently in such an organelle, it is important to select a promoter that functions in the organelle.

The so-called RNA sequencing method (RNA-Seq method), in which gene expression analysis is performed using a next-generation sequencer, is rapidly becoming widespread (for example, Non Patent Literature 2). By using the RNA-Seq method, gene expression can be analyzed with efficiency comparable to the efficiency of a microarray in terms of both cost and work. In addition, the expression analysis of lncRNA (long noncoding RNA) has also been performed using the RNA-Seq method (for example, Non Patent Literature 3). However, as far as the present inventors know, there is no known method capable of selecting a promoter that functions in an organelle and that even has desired activity intensity, by using the RNA-Seq method.

[Citation List]

[Non Patent Literature]

[NPL 1] Day, A. and Goldschmidt-Clermont, M., Plant Biotechnol. J., 9: 540 (2011)

[NPL 2] Mortazavi A., et al., Nat Methods. 5(7): 621-628 (2008)

[NPL 3] Di C., et al., Plant J., 80(5): 848-861 (2014)


[Summary of Invention]

[Technical Problem]

A problem to be solved by the present invention is thus to provide a method capable of selecting a promoter that functions in an organelle and has desired activity intensity, on a large scale using a next-generation sequencer, and to provide a promoter that functions in an organelle, which is obtained by the selection.

[Solution to Problem]

The present inventors have searched for a promoter capable of expressing a target protein, which is toxic in an organelle when highly expressed, in a large amount as stably as possible. Previously, reverse transcription PCR has been mainly used to detect organelle gene expression, so it has not been possible to compare the strength of promoters, where promoter screening methods are limited to qualitative classifications, such as high expression-type promoters or low expression and constitutive expression-type promoters. With the high expression-type promoters, however, the target protein exhibits its toxicity, which raises a problem of not being able to stably maintain the gene construct expressing the target protein. On the other hand, with the low expression and constitutive expression-type promoters, while the construct is stably maintained, there is a problem of not being able to expect large-scale expression of the target protein. The present inventors have thus come up with the idea that a promoter that can be stably maintained in the organelle and fully express the target protein may be obtained if the present inventors can select a promoter capable of achieving an expression level intermediate between the above two types of promoters from among the promoters present in organellar DNA.

In the selection of such a promoter, the present inventors have thought that by using the RNA sequencing method (RNA-Seq method), promoters satisfying the above conditions can be comprehensively screened. With the finding that in the RNA-Seq method for analyzing gene expression level, a sample prepared by isolating mRNA having a poly A sequence from mRNA is usually used, but the mRNA derived from organellar DNA does not have the poly A sequence added in the active form, the present inventors have decided to perform RNA-Seq using a sample comprising mRNA that does not have a poly A sequence and to use the data obtained thereby. Using the data, and with the amount of change in FPKM (fragments per kilobase of exon per million reads mapped) used as an index, the present inventors have selected promoters in which the amount of change is between the value at the high expression promoter and the value at the low expression promoter, thereby succeeded in identifying twelve promoters. The present inventors have further found that some of these promoters include sequences that cannot be predicted from the ORF information of the base sequence. As a result of further research based on these findings, the present inventors have achieved the completion of the present invention.

Specifically, the present invention is as follows.

[1] A method for selecting a promoter that functions in an organelle, the method comprising the following steps (1) to (5) :

(1) the step of preparing sequence information obtained by RNA sequencing analysis;
(2) the step of mapping the sequence information prepared in the step (1) onto a sequence of DNA of the organelle;
(3) the step of calculating the amount of change in RNA expression before and after in each region based on the mapping information obtained in the step (2);
(4) the step of selecting a region in which the amount of change obtained in the step (3) is within a range of preset reference values; and
(5) the step of identifying an upstream region of the region selected in the step (4) as a promoter functioning in the organelle.

[2] The method of [1], further comprising (6) the step of producing a construct including one or more promoters identified in the step (5) and verifying that the promoter functions in the organelle.
[3] The method of [1] or [2], wherein the amount of change in expression before and after in the region is the amount of change in FPKM.
[4] The method of any one of [1]-[3], wherein the organelle is a plastid or mitochondrion.
[5] A DNA having promoter activity in an organelle, the DNA comprising the following sequence (a), (b) or (c):

(a) a sequence set forth in any of SEQ ID NOs: 1 to 12;
(b) a sequence resulting from deletion, substitution or addition of one or more bases in the sequence (a); or
(c) a sequence having at least 90% identity with the sequence (a).

[6] A transformation vector comprising the sequence of [5].
[7] A cell having an organelle transformed with the transformation vector of [6].
[8] The cell of [7], wherein the organelle is a plastid or mitochondrion.
[9] The cell of [8], wherein the cell is a plant cell.
[10] A plant body having the plant cell of [9].

[Advantageous Effects of Invention]

According to the selection method of the present invention, promoters that function in organelles and have desired activity intensity can be selected on a large scale. Transformation of an organelle with the promoter obtained by such selection can produce cells expressing the target amount of protein.

[Brief Description of Drawings]

[Figure 1] Figure **1** shows a schematic diagram of a method of gene analysis by RNA-Seq. By gene analysis by RNA-Seq, the expression level of the gene can be quantified, and the position of the promoter can be specified.
[Figure 2] Figure **2** shows a map of a vector used in Examples. In the figure, the promoter region to be tested was inserted in the AtCpP03 part.
[Figure 3] Figure **3** shows the results of the relative activity of a promoter ten to eleven hours after the initiation of amplification using E. coli. The horizontal axis indicates the promoter SEQ ID NOs.

[Description of Embodiments]

<u>1. Method for selecting promoters that function in organelles</u>

The present invention provides a method for selecting a promoter that functions in an organelle (hereinafter, may be referred to as the "selection method of the present invention"). The selection method of the present invention includes the following steps (1) to (5):

(1) the step of preparing sequence information obtained by RNA sequencing (hereinafter, may be referred to as the "RNA-Seq") analysis;
(2) the step of mapping the sequence information prepared in the step (1) onto a sequence of organellar DNA;
(3) the step of calculating the amount of change in RNA expression in each region based on the mapping information obtained in the step (2);
(4) the step of selecting a region in which the amount of change obtained in the step (3) is within a range of preset reference values; and
(5) the step of identifying or determining an upstream region of the region selected in the step (4) as a promoter functioning in an organelle.

As used herein, the "region" encompasses, not only a single region, but also a group of regions consisting of a plurality of regions.
As shown in the Examples below, performing the above steps (1) to (5) demonstrated to be able to select promoters having a correlation with the function in the organelle. Therefore, while the selection method of the present invention is sufficient to include the steps (1) to (5), the selection method may further include a step of verifying that the promoter identified in the step (5) functions in an organelle. Accordingly, in one aspect, the selection method of the present invention includes (6) the step of producing a construct including one or more promoters identified in the step (5) and verifying that the promoter functions in an organelle.
As used herein, the "organelle" means organelles with DNA other than nuclear DNA, and examples of such organelles include, for example, plastids and mitochondria. Examples of the plastids include, for example, chloroplasts, etioplasts, amyloplasts, elaioplasts, chromoplasts and leucoplasts, and among them, preferable are chloroplasts, for which many transformation methods are known.
In the above step (1), as the sequence information to be prepared, for example, information published as a database (e.g., PRJNA213635 of Non Patent Literature 3) may be used, or information obtained by performing RNA-Seq using a sample comprising RNA that does not have a poly A sequence, prepared by a method known per se, may be used. Such a sample comprising RNA that does not have a poly A sequence can be prepared, for example, by extracting RNA from a sample, removing the nuclear rRNA sequence, fragmenting the RNA, synthesizing cDNA with a random primer or the like, and adding an aptamer sequence to the cDNA.
In the above step (2), mapping of organellar DNA onto a sequence can be performed using known mapping software (e.g., BWA, Bowtie, STAR, etc.). The organelle sequence can also be obtained from the database (for example, the sequence of Arabidopsis chloroplast genome is published as GenBank Accession No: NC_000932.1, and the sequence of Arabidopsis mitochondrial genome is published as GenBank Accession No: NC_001284.2). Alternatively, a DNA sequence may be newly identified by using next-generation sequencing or the like, and the sequence information thereof may be used.
In the above step (3), the expression of each gene can be calculated using, for example, FPKM, RPKM (which can be calculated by the below formula [Numeral 1]), TPM (transcripts per kilobase million), or the like. In addition, the amount of change in the expression of the RNA before and after in the region can be calculated using the value calculated above. FPKM (RPKM for single-end read) means a value obtained by correcting the read count data obtained from the RNA-Seq data by the total number of reads followed by correcting by the transcript length. TPM means a value indicating how many transcripts are present for a particular transcript when there are one million total transcripts in the sample. FPKM and RPKM can be calculated by the following formula ([Numeral 1]).

[Numeral 1]

$$FPKM_i = Y_i \frac{1000}{L_i} \frac{1000000}{N} = \frac{Y_i}{L_i N} 10^9$$

In the above formula, $FPKM_i$ represents the FPKM of the transcript i, N represents the total number of reads that have been mapped to the reference sequence, Yi represents the number of reads mapped to the region of the transcript **i** out of the total number of reads, and Li represents the length of the transcript **i.**

In addition, TPM can be calculated as follows. Assuming that $Y_t$ is the read count mapped to the transcript **t** and $L_t$ is the length of the transcript **t,** the number of reads per 1,000 bp of the transcript **t** can be calculated as follows ([Numeral 2]).

[Numeral 2]

$$T_t = \frac{Y_t}{L_t} 10^3$$

Subsequently, the total read count after correction by the transcript length is corrected to be one million. At this stage, the TPMt of the transcript **t** can be calculated as follows ([Numeral 3]).

[Numeral 3]

$$TPM_t = T_t \frac{1}{\sum_t T_t} 10^6$$

In the afore-mentioned step (4), the range of reference values can be set according to the desired intensity of promoter activity. For example, when the purpose is to select a promoter having an intermediate promoter activity between a promoter known to be a high expression-type promoter and a promoter known to be a low expression-type promoter, the amount of change in RNA expression in the promoter region and the region under its control can be calculated as the amount of change in the FPKM, RPKM, TPM, etc., and the range of the values thereof can be set as the range of the reference values. In one embodiment, for chloroplasts, the range of the reference values can be set to $10^1$ to $10^5$, preferably $10^2$ to $10^{4.5}$. Furthermore, for mitochondria, the range of the reference values can be set to 10 or more, preferably $10^{1.5}$ to $10^4$.

In the above step (5), upstream regions of genes etc. belonging within the reference value range of the step (4) can be identified as promoters that function in organelles. The above determination, or identification of the promoter region, can be performed by, for example, the below steps (i) to (iii), but the present invention is not limited to this method:

(i) predicting orf from the sequence information of the region;

(ii) if an increase in the FPKM value is observed near the start codon of the orf, checking to see if a sequence is present that is likely to be an SD (Shine-Dalgarno) sequence before the start codon, and if an SD sequence is present, selecting the SD sequence including the vicinity thereof as a candidate for the promoter region; if the orf is not found, or if the SD sequence is not found, selecting the sequence including the vicinity thereof in which the FPKM value has changed as a candidate for the promoter region, where the SD sequence refers to a common sequence found upstream of the start codon in prokaryotes, and where the sequence is known to bind ribosomes that translate mRNA into proteins and facilitate translation initiation; and

(iii) selecting an intergenic region as a candidate for the promoter region in order to prevent orf from entering the promoter sequence in a form containing the start codon.

Furthermore, the candidates may be narrowed down by using the ease of designing the PCR primer, or the presence or absence of the orf, as an index.

The above step (6) is capable of introducing, for example, the promoter identified in the step (5) and nucleic acids in which a gene (e.g., the reporter gene described in Section 2 below, etc.) is linked to the 3'side of the promoter sequence, into a cell to confirm whether the cell has desired activity using a method for measuring the expression level of the gene in the cell, etc. At this stage, while gene expression in the organelle may be detected or measured, cells having a prokaryotic transcriptional translation mechanism (e.g., E. coli, etc.), similarly to organelles, may be used. If no SD sequence is found in the promoter, it is preferable to add an SD sequence or the like (for example, the SD sequence of the T7g10 gene) as appropriate. Furthermore, the promoter identified by the selection method of the present invention can be appropriately included in the expression construct, and introduction of the expression vector into cells and verification as to whether the cells exhibit the desired expression activity allow selecting and identifying of an expression vector suitable for the promoter.

## 2. Promoters that function in organelles

In another embodiment, the present invention provides DNA that has promoter activity in an organelle, i.e., that functions as a promoter in the organelle, obtained by the selection method of the present invention (hereinafter, may be referred to as the "promoter DNA of the present invention"). The definition and type of organelle are as described in Section 1 above.

As shown in the Examples below, a sequence set forth in any of SEQ ID NOs: 1 to 12 was incorporated into a transformation vector to express a gene linked to a promoter in E. coli having a prokaryotic transcriptional translation mechanism similarly to an organelle. As a result, gene expression was confirmed so as to reflect the intensity of promoter activity predicted from the analysis result of RNA-Seq, and thus, all sequences set forth in any of SEQ ID NOs: 1-12 may have promoter activity in the organelle. Accordingly, in one aspect, the promoter DNA of the present invention comprises or consists of a sequence set forth in any of SEQ ID NOs: 1-12. SEQ ID NO: 1 is the sequence of the region from the 66967th base to the 66739th base in the sequence of Arabidopsis chloroplast genome (GenBank Accession No: NC_000932.1). SEQ ID NO: 2 is the sequence of the region from the 1467th base to the 1574th base in the above sequence. SEQ ID NO: 3 is the sequence of the region from the 54960th base to the 54593rd base in the above sequence. SEQ ID NO: 4 is the sequence of the region from the 34250th base to the 34084th base in the above sequence. SEQ ID NO: 5 is the sequence of the region from the 13505th base to the 13608th base in the above sequence. SEQ ID NO: 6 is the sequence of the region from the 64320th base to the 64558th base in the above sequence. SEQ ID NO: 7 is the sequence of the region from the 41893rd base to the 42081st base in the above sequence. SEQ ID NO: 8 is the sequence of the region from the 112600th base to the 112806th base in the above sequence. SEQ ID NO: 9 is the sequence of the region from the 32636th base to the 32390th base in the above sequence. SEQ ID NO: 10 is the sequence of the region from the 14768th base to the 14889th base in the above sequence. SEQ ID NO: 11 is the sequence of the region from the 232031st base to the 231696th base in the sequence of Arabidopsis mitochondrial genome (GenBank Accession No: NC_001284.2). SEQ ID NO: 12 is the sequence of the region from the 206221st base to the 206059th base in the above sequence.

In another aspect, the promoter DNA of the present invention includes, or consists of, a sequence resulting from deletion, substitution or addition of one or more (e.g., 2, 3, 4, 5, 6, 7, 8, 9, 10, 11 or 12) bases in a sequence set forth in any of SEQ ID NOs: 1 to 12, and the promoter DNA has promoter activity in an organelle.

In still another aspect, the promoter DNA of the present invention includes, or consists of, a sequence having at least 90% identity (e.g., 91%, 92%, 93%, 94%, 95%, 96%, 97%, 98%, 99% or more) (however, except for 100%) with a sequence set forth in any of SEQ ID NOs: 1 to 12, and the promoter DNA has promoter activity in an organelle.

Base sequence identity can be calculated using NCBI BLAST (National Center for Biotechnology Information Basic Local Alignment Search Tool) (https://blast.ncbi.nlm.nih.gov/Blast.cgi), a homology calculation algorithm, under the condition that various parameters are set to default values.

The promoter DNA of the present invention having the above-mentioned sequence may have promoter activity similarly even with any other sequences included therein. Thus, the length of the promoter DNA of the present invention is, without particular limitation, preferably 500 bases or less, and more preferably 400 bases or less.

The method for synthesizing the promoter DNA of the present invention is not particularly limited, and conventionally known methods can be adopted. Examples of the synthesis method include a synthesis method by a genetic engineering approach, a chemical synthesis method, and the like. Examples of the genetic engineering approach include an in vitro transcription synthesis method, a method using a vector, and a method with a PCR cassette. Examples of the vector include, without particular limitation, non-viral vectors, such as plasmids, and viral vectors. Examples of the chemical synthesis method include, without particular limitation, a phosphoramidite method and an H-phosphonate method, for example. For the chemical synthesis method, a commercially available automatic nucleic acid synthesizer, for example, can be used. For the chemical synthesis method, amidite is used in general. The amidite is not particularly limited, and examples of commercially available amidite include, for example, ACE amidite, TOM amidite, CEE amidite, CEM amidite and TEM amidite.

Confirmation that the DNA has the desired promoter activity in an organelle can be performed by the method described in Section 1 above.

## 3. Transformation vectors including the promoter of the present invention

The present invention also provides a transformation vector including the promoter of the present invention described in Section 2 above (hereinafter, may be referred to as the "vector of the present invention"). In the vector of the present invention, the promoter of the present invention is operably linked to a gene (hereinafter, may be referred to as the "target gene") encoding a protein or ribonucleic acid of interest (e.g., non-coding RNA).

Even proteins harmful in the cytoplasm can be accumulated in organelles; thus, the target gene is not particularly limited, and examples thereof include, for example, merA genes, pest resistant genes and herbicide resistant genes, genes involved in biosynthesis of carotenoids and vitamin E, which have an antioxidant effect, and genes associated with photosynthesis, such as fructose 1,6-bisphosphatase/sedoheptulose 1,7-bisphosphatase genes. When the vector of the present invention has a metallothionein gene, such as the merA gene, in an organelle, the cells into which the vector has been introduced can acquire resistance to harmful heavy metals and mercury in the environment such as soil. When the vector of the present invention has a pest resistant gene or an antibiotic resistant gene (aadA), the cells into which the vector has been introduced can acquire pest resistance and resistance to infectious bacteria. When the vector of the present invention has a gene involved in biosynthesis of carotenoids and vitamin E having an antioxidant effect, the cells into which the vector has been introduced can improve the content of useful components, such as carotenoids and vitamin E, in the fats and oils generated by the cells. Furthermore, when the vector of the present invention has a fructose 1,6-bisphosphatase/sedoheptulose 1,7-bisphosphatase gene, derived from cyanobacteria and associated with photosynthesis, the cells into which the vector has been introduced can be taller, have a larger leaf area, grow faster, and have an increased ability to synthesize sugars and starches, as compared with the wild-type strain.

Expression of harmful proteins (such as CMS causative proteins) in organelles inhibits pollen development and results in cytoplasmic male sterility (CMS); however, fertilization is possible when pollen of another pollen parent is provided. Thus, this phenomenon is also used for seed production of F1 crops. Examples of the target gene include, without particular limitation, CMS causative genes, for example, such as orf79 genes, orfH79 genes, orf284 genes, orf352 genes, urf13 genes, orf355 genes, atp6-C genes, orf256 genes, orf260 genes, orf138 genes, orf125 genes, orf224 genes, orf222 genes, orf463 genes, orfH522 genes, orf107 genes, preSatp6 genes, orf129 genes, G cox2 genes, pcf genes, orf239 genes, Ψatp6-2 genes and orf456 genes. When the vector of the present invention has a CMS causative gene etc., the individual into which the vector has been introduced cannot self-fertilize due to inhibition of germ cell development, and thus can easily cross-pollinate to form F1 seeds. In addition, if an individual having an appropriate fertility recovery factor is used, an individual keeping the target gene in the organelle can be maintained.

Even ribonucleic acids that undergo host-specific and unique metabolism in the cytoplasm can be accumulated in organelles in their transcribed form; thus, the target gene is not particularly limited, and examples thereof include, for example, constructs that express target pest-specific conserved sequences included in genes essential for pests, such as acetylcholinesterase genes, chitin synthase genes and actin genes of pests, whereby the expressed conserved sequences can form double-stranded ribonucleic acids. When the vector of the present invention includes a construct that expresses a pest-specific conserved region which results in a double-stranded ribonucleic acid, it can be lethal for the pests that have eaten cells which have been introduced the vector and thus this confers the cells repellent effects. This is because double-stranded ribonucleic acids that do not undergo plant-specific metabolism produce RNAi by independently metabolizing ribonucleic acids in the pest, and the expression of essential genes is suppressed.

It is preferable to add a sequence homologous to or highly identical to the gene (e.g., trnG (tRNA-Gly (GCC)), trnV (tRNA-Val (GAC)), trnfM (tRNA-fMet (CAU)), rbcL gene, accD gene, trnI (tRNA-Ile (GAU)) and trnA (tRNA- Ala (UGU)), 3'rps12 (ribosomal protein S12 exon-3) gene, trnV (tRNA-Val (GAC)), etc.) sequence of organellar DNA to the vector of the present invention, on the 5' and 3'sides of the gene to be transduced, so as to be introduced into organellar DNA by homologous recombination. Examples of the length of the homologous sequence include, for example, a base length of about 500 to 1500. The homologous sequence can be appropriately designed based on the base sequence information of known organellar DNA, etc.

The vector of the present invention preferably has a gene described in a codon table corresponding to the organelle to be expressed. In vertebrates for example, the UGA codon in nuclear expression is a stop codon, whereas it encodes tryptophan in mitochondria. In accordance with such a difference in the codon table, it is preferable to make a control so that an appropriate protein sequence can be expressed only in the appropriate organelle.

The vector of the present invention preferably has a marker gene for selecting transformants. Examples of the marker gene include reporter genes (e.g., genes encoding fluorescent protein, genes encoding luminescent protein, genes encoding protein that assists fluorescence, luminescence or color development), drug resistant genes, and the like. Only one type of marker gene may be used, or two or more types thereof (for example, a gene encoding a fluorescent protein in combination with a drug resistant gene) may be used. Herein, a protein encoded by a marker gene may be referred to as a "marker protein".

Examples of the fluorescent protein include, but are not limited to: for example, blue fluorescent proteins such as Sirius, TagBFP and EBFP; cyan fluorescent proteins such as mTurquoise, TagCFP, AmCyan, mTFP1, MidoriishiCyan and CFP; green fluorescent proteins such as TurboGFP, AcGFP, TagGFP, Azami-Green (e.g., hmAG1), ZsGreen, EmGFP, EGFP, GFP2 and HyPer; yellow fluorescent proteins such as TagYFP, EYFP, Venus, YFP, PhiYFP, PhiYFP-m, TurboYFP, ZsYellow and mBanana; orange fluorescent proteins such as Kusabira Orange (e.g., hmKO2) and mOrange; red fluorescent proteins such as TurboRFP, DsRed-Express, DsRed2, TagRFP, DsRed-Monomer, AsRed2 and mStrawberry; and near-infrared fluorescent proteins such as TurboFP602, mRFP1, JRed, KillerRed, mCherry, HcRed, KeimaRed (e.g., hdKeimaRed), mRasberry and mPlum.

Examples of the luminescent protein include, but are not limited to, Aequorin. Furthermore, examples of the protein that assists fluorescence, luminescence or color development include, but are not limited to, enzymes that decompose a fluorescent, luminescent, or color development precursor such as a luciferase, phosphatase, peroxidase, or $\beta$-lactamase.

Examples of the drug resistant genes include, for example, drug resistant genes such as hygromycin-resistant genes (hygromycin phosphotransferase gene, hpt), spectinomycin resistance genes (aadA), kanamycin-resistant genes and neomycin-resistant genes, and herbicide resistant genes such as ALS (AHAS) genes and PPO genes.

It is preferable that an enhancer, a promoter, a leader and/or a terminator sequence for controlling the expression of the marker gene is included upstream or downstream of the marker gene, respectively. Moreover, for organelles, the gene may be plural and form an operon sequence. Examples of the promoter include, for example, rrn promoters, cox2 promoters, psbA promoters, accD promoters, clpP promoters, atpB promoters, rpl32 promoters, H strand promoters, promoters of the elongation factor 1$\alpha$ gene (EF1$\alpha$ promoters), 35S promoters, PPDK promoters, PsPAL1 promoters, PAL promoters and UBIZM1 ubiquitin promoters. Among them, organelle-derived promoters are preferred. Examples of the leader sequence include, for example, cry9Aa2 leaders and atpB leaders. When the expression of a plurality of genes is induced with one promoter, it is preferable to include a leader sequence. Examples of the terminator include, for example, rbcL terminators, rps16 terminators, CaMV35S terminators, ORF25polyA transfer terminators and psbA terminators.

The vector of the present invention can also have a nucleic acid encoding an organelle transition signal peptide. The mitochondrial translocation signal peptide usually consists of a pattern in which two or three hydrophobic amino acids and basic amino acids appear alternately, and examples thereof include, for example, MLSLRQSIRFFK (SEQ ID NO: 37). The chloroplast translocation signal peptide usually consists of a sequence of several tens to hundreds of amino acid residues rich in serine and threonine and poor in acidic amino acids (Javis P. and Lopez-Juez E., Nat. Rev. Mol. Cell Biolo., 14, 787-802 (2013)). Examples of the peroxisome transfer peptide include C-terminal SKL.

The vector of the present invention can be constructed, for example, by introducing a DNA in which a target gene is linked to the promoter of the present invention, and if necessary, the above-mentioned marker gene or the like, into an appropriate vector. Specifically, examples thereof include, but are not limited to, for example, the method described in Svab et al., Proc. Nal. Acad. Sci. USA, 87, 8526 (1990), the method described in Sikdar et al., Plant Cell Rep., 18, 20 (1998), the method described in Sidorov et al.: Plant J., 19, 209 (1999), the method described in Ruf, S. et al., Nature biotechnol., 19, 870 (2001), and the method described in Hou et al., Transgenic Res., 12, 111 (2003). In addition, such a vector can be prepared based on a pBI-based vector, a pUC-based vector, a pPZP-based vector (Hajdukiewicz P., et al., Plant Mol Biol., 25: 989-94, (1994)), a pCAMBIA-based vector, a pSMA-based vector, or the like, which can introduce a target gene into a plant via Agrobacterium. Examples of the pBI-based binary vector include, for example, pBI121, pBI101, pBI101.2, and pBI101.3. Examples of the pUC-based vector include, for example, pUC18, pUC19 and pUC9. In addition, plant virus vectors such as cauliflower mosaic virus (CaMV), bean golden mosaic virus (BGMV) and tobacco mosaic virus (TMV) may also be used.

Examples of a method for inserting the promoter of the present invention into a vector include, for example, a method of leaving DNA containing the promoter or the like with an appropriate restriction enzyme, followed by insertion into a restriction enzyme site or multicloning site of the vector and ligation to the vector.

## 4. Cells with organelle transformed by the vector of the present invention

Introducing the transformation vector prepared as described in Section 3 above into a host cell can produce a cell having a transformed organelle (hereinafter, may be referred to as the "cell of the present invention").

The host cell may be any cell having an organelle, and examples thereof include, for example, plant cells, animal cells, microorganisms, insect cells, etc., where plant cells are preferable. The plants from which the plant cells are derived may be any plants; however, monocotyledonous plants or dicotyledonous plants are preferable. Examples of the monocotyledonous plants include, but are not limited to, gramineous plants and amaryllidaceae plants. The gramineous plants include plants belonging to Oryza, Triticum, Hordeum, Secale, Saccharum, Sorghum or Zea, which specifically include, but are not limited to, maize, sorghum, wheat, rice, oat, barley, rye, and millet. Preferable gramineous plants are maize, wheat, and rice. Examples of the amaryllidaceae plants include, but are not limited to, onions, leeks and garlic. Preferable amaryllidaceae plants are onions and leeks.

Examples of the dicotyledonous plants include, but are not limited to, Brassicaceae plants, Leguminosae plants, Solanaceae plants, Cucurbitaceae plants, Convolvulaceae plants and Asteraceae plants. Examples of the Brassicaceae plants include plants belonging to Raphanus, Brassica, Arabidopsis, Wasabia, or Capsella, which specifically include, but are not limited to, Brassica rapa var. pekinensis, rapeseed, cabbage, cauliflower, Raphanus sativus var. hortensis, Brassica rapa subsp. oleifera, Arabidopsis thaliana, Eutrema japonicum, and Capsella bursa-pastoris. Preferable cruciferous plants are Arabidopsis thaliana, Brassica rapa var. pekinensis, and rapeseed. Examples of the Leguminosae plants include, but are not limited to, for example, soybeans, Vigna angularis, Phaseolus vulgaris, and Vigna unguiculata. Preferable Leguminosae plants are soybeans and Phaseolus vulgaris. Examples of the Solanaceae plants include, but are not limited to, for example, tobacco, tomato, petunia, eggplant and potato. Preferable Solanaceae plants are tobacco, tomato and petunia. Examples of the Cucurbitaceae plants include, but are not limited to, for example, oriental melon, cucumber, melon and watermelon. Preferable Cucurbitaceae plants are oriental melon. Examples of the Asteraceae plants include, but are not limited to, for example, sunflower, chrysanthemum and dandelion. Preferable Asteraceae plants are sunflower. Examples of the Convolvulaceae plants include, but are not limited to, for example, morning glory, sweet potato, and bindweed. Preferable Convolvulaceae plants are sweet potato.

Apart from the above plants, the examples of the plants further include plants such as Rosaceae, Lamiaceae, Liliaceae, Chenopodiaceae, Polygonaceae, Amaranthaceae, Apiaceae and Araceae, and additionally, any tree species, any fruit tree species, Moraceae plants (e.g., rubber), and Malvaceae plants (e.g., cotton).

Examples of the animal cells include, for example, cell lines such as monkey COS-7 cells, monkey Vero cells, Chinese hamster ovary (CHO) cells, dhfr gene-deficient CHO cells, mouse L cells, mouse AtT-20 cells, mouse myeloma cells, rat GH3 cells and human FL cells; pluripotent stem cells such as human and other mammalian iPS cells and ES cells; primary cultured cells prepared from various tissues, etc. Examples of the microorganisms include, for example, yeast (e.g., Saccharomyces cerevisiae, AH22, AH22R-, NA87-11A, DKD-5D, 20B-12, Schizosaccharomyces pombe NCYC1913, NCYC2036, Pichia pastoris KM71, etc.), Chlamydomonas (e.g., Chlamydomonas reinhardtii), etc. Examples of the insect cells include, for example, established cells derived from Spodoptera frugiperda larva (Spodoptera frugiperda cell; Sf cell), MG1 cells derived from the midgut of Trichoplusia ni, High Five™ cells derived from Trichoplusia ni eggs, cells derived from Mamestra brassicae, cells derived from Estigmena acrea, established cells derived from silkworm (Bombyx mori N cell; BmN cell), etc.

The method for transformation by introducing the vector of the present invention into a host cell includes, for example, known methods such as the particle gun method (Svab Z., et al., Proc Natl acad Sci USA, 87: 8526-8530 (1990)), PEG method (Golds T., et al., Bio Technol., 11:95-97 (1992)) and electroporation method, the methods of which can be preferably used. For example, in the particle gun method, a vector can be introduced into a host cell by sprinkling the vector on extremely fine particles of gold or tungsten and driving the particles, to which the vector is attached, into the host cell with an explosive or a high-pressure gas. The definition and type of organelle are as described in Section 1 above, but plastids and mitochondria are preferable, and among others, chloroplasts and mitochondria are more preferable. In addition, as described in Chuah J.A., et al., Scientific Reports, 5: 7751 (2015), it is also preferable to introduce the vector of the present invention into an organelle using a complex of an organelle transfer signal peptide and a cell membrane penetrating peptide.

When the organelle in the cell is in a heteroplasmic state, a homoplasmic individual may be prepared by a method known per se. Examples of the above method known per se include, for example, methods of obtaining homoplasmic individuals by repeating selection culture two to three times. In addition, as described in WO 2013/077420 A1, it is also possible to promote homoplasmicization by introducing nucleic acids encoding an organelle-migrating lethal protein (e.g., barnase) into nuclei, introducing nucleic acids encoding the protein inhibitor into organelles, and selectively eliminating only organelles that do not have the nucleic acids encoding the inhibitor.

After successful achievement of organelle transformation and, if necessary, achievement of homoplasmicization, exogenous nucleic acids incorporated into the organelles by the introduction of the vector of the present invention, may be removed. Examples of means for removing the nucleic acids incorporated in the organelle include a method using a Cre-loxP system or a FLP-FRT system, a method using a transposon, and a natural removal method via an endogenous homologous recombination activity, etc.

The target protein can be manufactured by culturing the cell of the present invention or a plant body having the cell. As used herein, the "plant body" encompasses all of plant individual, a plant organ, a plant tissue, a plant cell, and a seed. Examples of the plant organ include a root, a leaf, a stem, and a flower and the like. Furthermore, the plant cell also includes a cell in a plant body in addition to a cultured cell. Furthermore, the plant cell in various forms (e.g., a suspension cultured cell, a protoplast, a section of a leaf, a section of a root, a callus, an immature embryo, pollen and the like) is included.

Culture of the cells of the present invention can be implemented in accordance with a known method in accordance with the type thereof. A preferable medium used for culture is a solid medium (e.g., an agar medium, an agarose medium, a gellan gum medium or the like). Furthermore, a medium preferably contains a carbon source, a nitrogen source, an inorganic substance or the like which is necessary for growth of a transformant. When the cell of the present invention is a plant cell, used as a basal medium is, for example, an N6 medium, an MS medium, an LS medium, a B5 medium or the like. A plant growth substance (e.g., auxins, cytokinins or the like) or the like may be appropriately added to a medium. pH of a medium is preferably about 5 to about 8. Culture temperature can be appropriately selected within the range of about 20°C to about 35°C in general depending on the type of the plant cells. For example, a rice callus can be generally cultured at 28 to 33°C, and preferably at 30 to 33°C.

Examples of the medium used for culturing animal cells include, for example, a minimum essential medium (MEM) containing about 5 to about 20% fetal bovine serum [Science, 122, 501 (1952)], Dulbecco's Modified Eagle's Medium (DMEM) [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. The pH of the medium is preferably about 6 to about 8. Culturing is usually carried out at about 30°C to about 40°C. If necessary, ventilation or stirring may be performed.

Examples of the medium for culturing microorganisms include, for example, Burkholder minimum medium [Proc. Natl. Acad. Sci. USA, 77, 4505 (1980)], SD medium containing 0.5% casamino acid [Proc. Natl. Acad. Sci. USA, 81, 5330 (1984)], etc. The pH of the medium is preferably about 5 to about 8. Culturing is usually carried out at about 20°C to about 35°C. If necessary, ventilation or stirring may be performed.

Examples of the medium used for culturing insect cells or insects include, for example, Grace's Insect Medium [Nature, 195, 788 (1962)] with appropriate additives, such as inactivated 10% bovine serum, added thereto, etc. The pH of the medium is preferably from about 6.2 to about 6.4. Culturing is usually carried out at about 27°C. If necessary, ventilation or stirring may be performed.

In addition, the medium may contain saccharides as a carbon source, vitamins, supports for solidifying the medium, and the like. Examples of the saccharides include, for example, glucose, sucrose, etc. The amount of the saccharides added is about 1 to 10% by weight, preferably about 2 to 5% by weight. Examples of the vitamins include, for example, thiamine hydrochloride, pyridoxine hydrochloride, nicotinic acid, inositol or the like. Examples of the supports include, for example, agar, gellan gum or paper bridges. In addition, the medium may include amino acids (e.g., glycine, etc.), adenine, coconut water, and the like.

When the cell of the present invention is a callus, the callus can be regenerated into a plant body by a regeneration method known per se. For rice, examples of the regeneration method include a method described in Toki S. et al., Plant Physiol. 100(3):1503-1507 (1992), a method described in Christou P. et al., Bio/Technology 9:957-962 (1991), a method described in Hiei Y. et al., Plant J. , 6 : 271-282, (1994) and the like.

Once a plant body comprising cells with transformed organelles included therein is obtained in this manner, it is possible to obtain progeny from the plant body by sexual reproduction or asexual reproduction. Furthermore, it is also possible to obtain a propagation material (e.g., seeds, fruits, cuttings, stubbles, calli, protoplasts or the like) from the plant body or progeny or clones thereof and mass-produce the plant body based on the propagation material.

The target protein can be isolated by extracting and purifying useful components from the cells cultured as described above by a method known per se, or by secreting the target protein in the culture medium and recovering the target protein. Alternatively, freezing and drying processes may be performed on the cells, and the cells may be used as they are. Similarly, the protein can be isolated by extracting and purifying useful components from the above plant body by a method known per se. Alternatively, the plant body may be used as it is. Examples of the method known per se include, for example, a method of crushing the plant body with a mixer or mortar, followed by immersing in water or saline, and removing the crushed residue by centrifugation or filtration.

Hereinafter, the present invention will be described with reference to Examples. Note, however, that the present invention is not limited to these examples.

[Examples]

## Example 1: identification of organelle promoter

<Analysis of next-generation sequence data>

For the analysis of the next-generation sequence data, the data of Non Patent Literature 3 (PRJNA213635) was used, where RNA extracted from Arabidopsis thaliana was pretreated with Ribo minus RNA-seq and sequenced using HiSeq 2000 by Illumina, Inc. FastQC was used for the quality checking of the sequence. In addition, BWA was used for the mapping on the Arabidopsis chloroplast genome (NC_000932.1) and the Arabidopsis mitochondrial genome (NC_001284.2). Based on this mapping information, FPKM (reads per kilobase of exon per million mapped) was calculated for each region using igv (integrative genomics viewer), and the first half of the region where the change in FPKM was large was estimated to be the promoter sequence of the organelle.

<Construction of plasmid>

For testing promoter activity in chloroplasts in E. coli and plants, PKKP23 (Figure 2) was used, which is capable of expressing the spectinomycin resistance gene (aadA) and red fluorescent protein (TagRFP) with rrn, a known potent organelle promoter, and which is capable of expressing the green fluorescent protein (GFP) with the promoter sequence to be tested. The pKKP23 was constructed for each promoter to be tested, with each component amplified by the PCR method and using the NEBuilder HiFi DNA Assembly Master Kit (NEB). Table 1 shows the primers used in the PCR method.

[Table 1]

| Base Sequence (5' →3' ) | Primer Name | SEQ ID NO |
|---|---|---|
| gtgtgatttgtttagttggga | AtCpP10-F | 13 |
| CATATtgccctctgacagaaataagaac | AtCpP10-R | 14 |
| ttgcttttcaaagatttatgaaggatta | AtCpP08-F | 15 |
| gaa agg gat gat cca tga ata ttg ata tgt | AtCpP08-R | 16 |
| cegttgageacectatggatatgtc | AtCpP07-F | 17 |
| aacccgccaacagtcactca | AtCpP07-R | 18 |
| ctttatctgaataatgagtcatccga | AtCpP09-F | 19 |
| ggcattttcagggcgctcaa | AtCpP09-R | 20 |
| gacacggttatacatcgacaagcaag | AtCpP06-F | 21 |
| CATACtgaactccagatattctcgtagggaatcg | AtCpP06-R | 22 |
| ggctggattaatcttagcga | AtCpP05-F | 23 |
| CATgataagttectcacacca | AtCpP05-R | 24 |
| ctgcagttttgggctttggc | AtCpP04-F | 25 |
| gaatactagaagaaaggcacctacacc | AtCpP04-R | 26 |
| ggcctttacgttttcaaatggaatcg | AtCpP01-F | 27 |
| Tac egg tgc tac gga aaga | AtCpP01-R | 28 |
| tccattttacattggttgacatggct | AtCpP02-F | 29 |
| ataatcagggactcccaagcgca | AtCpP02-R | 30 |
| catggatgaattccgcatattgtcatatct | AtCpP03-F | 31 |
| CATAAgtccctccctacaagtca | AtCpP03-R | 32 |
| aaacatgtgggcgcaaaa | AtMtP01-F | 33 |
| aagatgcacggttccagtc | AtMtP01-R | 34 |
| tctagttagtagactcagaaaggcattgtat | AtMtP02-F | 35 |
| aacccaagcgagcattcaaatatct | AtMtP02-R | 36 |

Results

Based on the data of Non Patent Literature 3, which is about an analysis of Arabidopsis RNA using Ribo minus RNA-seq, the transcription initiation position and expression level were estimated from FPKM. In addition, the upstream region at the point where FPKM increases was predicted to be the promoter region in organelles. Specifically, (i) the orf was predicted from the sequence information of the region; (ii) when an increase in the FPKM value was observed near the start codon of orf, it was confirmed whether there was a sequence likely to be an SD (Shine-Dalgarno) sequence before the start codon; and if such an SD sequence was present, the SD sequence including the vicinity thereof was selected as a candidate for the promoter region. If the orf is not found, or if the SD sequence is not found, the sequence including the vicinity thereof, in which the FPKM value changed, was selected as a candidate for the promoter region. (iii) The intergenic region was selected as a candidate for the promoter region in order to prevent the orf from entering the promoter sequence in a form containing the start codon. Furthermore, the candidates were narrowed down by using the ease of designing the promoter and the presence or absence of the orf as indicators. As a result, the twelve regions shown in Table 2 were selected as promoter sequences estimated to have different expression levels. "Expected Intensity" in the table indicates the amount of change in the FPKM value before and after in the region.

[Table 2]

| Region Name | Original Sequence | Position | Downstream o r f | Expected Intensity | SEQ ID NO |
|---|---|---|---|---|---|
| CpP01 | NC_000932. 1 | 66967-66739 | psaJ | $10^{4}$ | 1 |
| CpP02 | NC_000932. 1 | 1467-1574 | psbA | $10^{3-4.5}$ | 2 |
| CpP03 | NC_000932. 1 | 54960-54593 | rbcL | $10^{4.5}$ | 3 |
| CpP04 | NC_000932. 1 | 34250-34084 | (pscC) | $10^{4}$ | 4 |
| CpP05 | NC_000932. 1 | 13505-13608 | atpH | $10^{3}$ | 5 |
| CpP06 | NC_000932. 1 | 64320-64558 | psbE | $10^{2}$ | 6 |
| CpP07 | NC_000932. 1 | 41893-42081 | psaA | $10^{2}$ | 7 |
| CpP08 | NC_000932. 1 | 112600-112806 | ndhF | $10^{1-2}$ | 8 |
| CpP09 | NC_000932. 1 | 32636-32390 | psbD | $10^{2-4}$ | 9 |
| CpP10 | NC_000932. 1 | 14768-14889 | atpI | $10^{2}$ | 10 |
| MtP01 | NC_001284.2 | 232031-231696 | none | $10^{2}$ | 11 |
| MtP02 | NC_001284.2 | 206221-206059 | none | 101.5 | 12 |

Example 2: Analysis of organelle promoter activity

<Measurement of promoter activity in E. coli>

For the measurement of the activity of each promoter in E. coli, pKKP23 for each promoter to be tested was prepared and transformed into E. coli JM109 strain. As the inoculum solution, a culture solution cultured with shaking overnight in the presence of antibiotics was used. This bacterial solution was inoculated into an antibiotic-containing LB medium dispensed into a 96-well plate and subjected to rotary culture at 37°C. The growth curve of each E. coli strain under such circumstances was measured by EPOCH2 (BioTek). In addition, the fluorescence values of GFP and RFP were measured with a plate reader at each time after inoculation. SectraMax Paradigm (Moleculu Devises) or GloMax (Promega) was used as the plate reader. GFP/RFP was calculated as the activity value of each promoter, and the relative value with the promoter sequence showing the highest activity was calculated. Figure **3** shows the results.

<Measurement of promoter activity in plants>

For the measurement of the activity of each promoter in the chloroplast of the plant, the plasmids used for the measurement of the promoter activity in E. coli are used respectively. As a plant, young leaves of tobacco (Nicotiana tabacum cv. Petit Havana) are used, and DNA-coated gold particles are introduced by the particle gun method using a PDS-1000/He (Bio-Rad) equipped with a Hepta adapter (Bio-Rad). For how to use the device, the Bio-Rad protocol is used, and selection of transformed plants is performed according to the methods described in Pal Maliga and Tarinee Tungsuchat-Hung (Methods Mol Biol. 1132: 205-220, (2014)). Individuals with transformed chloroplasts are selected with spectinomycin 500 mg/L, and the amount of GFP per total protein in young leaves is calculated as the activity value of each promoter.

Results

Like organelles, E. coli has a prokaryotic transcriptional translation mechanism; thus, the promoter activity of these regions was confirmed from the expression level of each protein in E. coli. When GFP was inserted downstream of the estimated promoter sequence, GFP expression was observed in correlation with promoter strength. From this, it was revealed that the promoter functions in the organelle. In addition, surprisingly, it was found that P04, P11, and P12 have promoter activity even though the promoter region was set based only on the RNA-seq data, not based on the prediction result of the ORF.

[Industrial Applicability]

According to the present invention, promoters that function in organelles and have the desired activity intensity can be selected on a large scale. By transforming an organelle using the promoter obtained by such selection, such cells can be prepared that can stably express a sufficient level of target protein that may adversely affect the host at the maximum expression level thereof.

The present application is on the basis of Japanese Patent Application No. 2019-042535 filed in Japan (filing date: March 8, 2019), the entire contents of which is incorporated herein.

## Claims

**1.** A method for selecting a promoter that functions in an organelle, the method comprising the following steps (1) to (5) :

(1) the step of preparing sequence information obtained by RNA sequencing analysis;
(2) the step of mapping the sequence information prepared in the step (1) onto a sequence of DNA of the organelle;

(3) the step of calculating the amount of change in RNA expression before and after in each region based on the mapping information obtained in the step (2);
(4) the step of selecting a region in which the amount of change obtained in the step (3) is within a range of preset reference values; and
(5) the step of identifying an upstream region of the region selected in the step (4) as a promoter functioning in the organelle.

**2.** The method of claim 1, further comprising (6) the step of producing a construct including one or more promoters identified in the step (5) and verifying that the promoter functions in the organelle.

**3.** The method of claim 1 or 2, wherein the amount of change in expression before and after in the region is the amount of change in FPKM.

**4.** The method of any one of claims 1-3, wherein the organelle is a plastid or mitochondrion.

**5.** A DNA having promoter activity in an organelle, the DNA comprising the following sequence (a), (b) or (c):

(a) a sequence set forth in any of SEQ ID NOs: 1 to 12;
(b) a sequence resulting from deletion, substitution or addition of one or more bases in the sequence (a); or
(c) a sequence having at least 90% identity with the sequence (a).

**6.** A transformation vector comprising the sequence of claim 5.

**7.** A cell having an organelle transformed with the transformation vector of claim 6.

**8.** The cell of claim 7, wherein the organelle is a plastid or mitochondrion.

**9.** The cell of claim 8, wherein the cell is a plant cell.

**10.** A plant body having the plant cell of claim 9.

Fig. 1

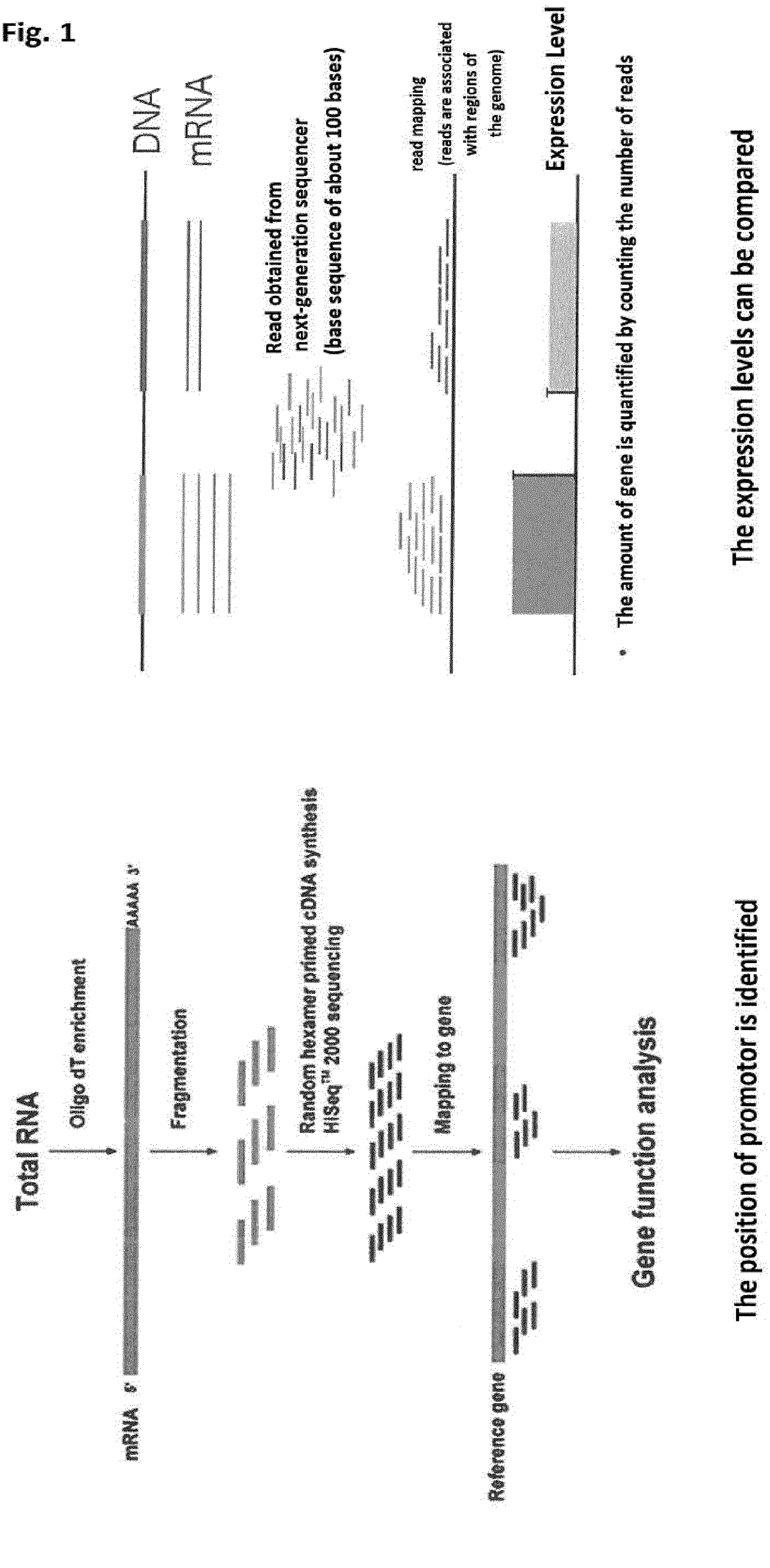
DNA
mRNA
Read obtained from next-generation sequencer (base sequence of about 100 bases)
read mapping (reads are associated with regions of the genome)
Expression Level
• The amount of gene is quantified by counting the number of reads
The expression levels can be compared
Total RNA
Oligo dT enrichment
mRNA 5'
AAAAA 3'
Fragmentation
Random hexamer primed cDNA synthesis HiSeq™ 2000 sequencing
Mapping to gene
Reference gene
Gene function analysis
The position of promotor is identified

Fig. 2

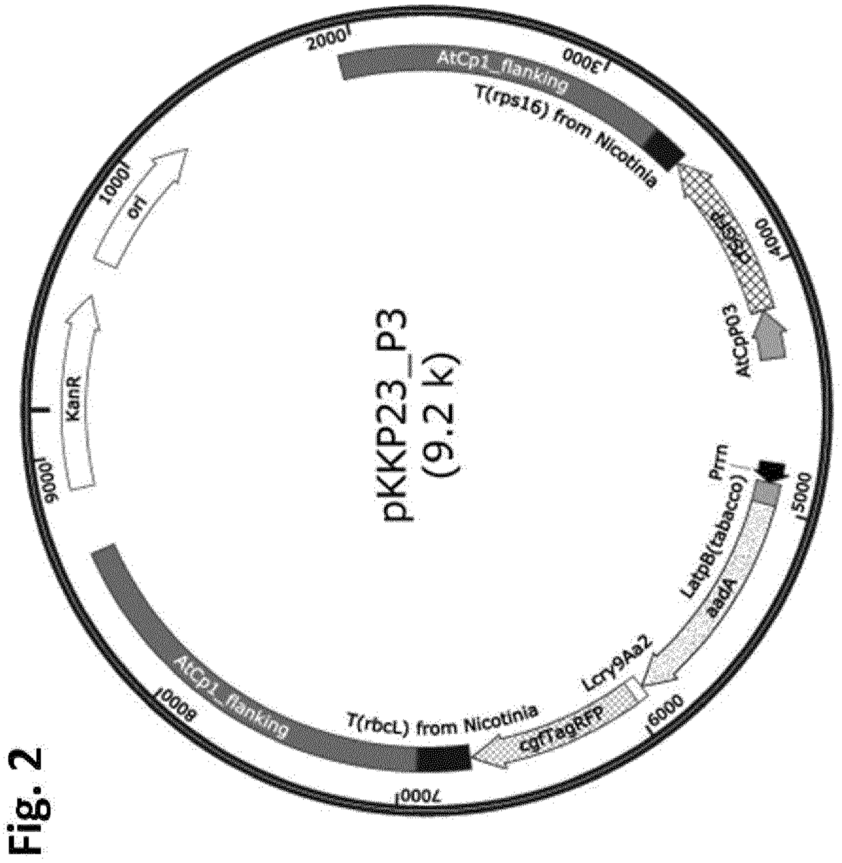
pKKP23_P3
(9.2 k)
AtCp1_flanking
T(rps16) from Nicotinia
AtCpP03
Prrn
LatpB(tabacco)
aadA
Lcry9Aa2
cgfTagRFP
T(rbcL) from Nicotinia
AtCp1_flanking
KanR
ori
1000
2000
3000
4000
5000
6000
7000
8000
9000

Fig. 3

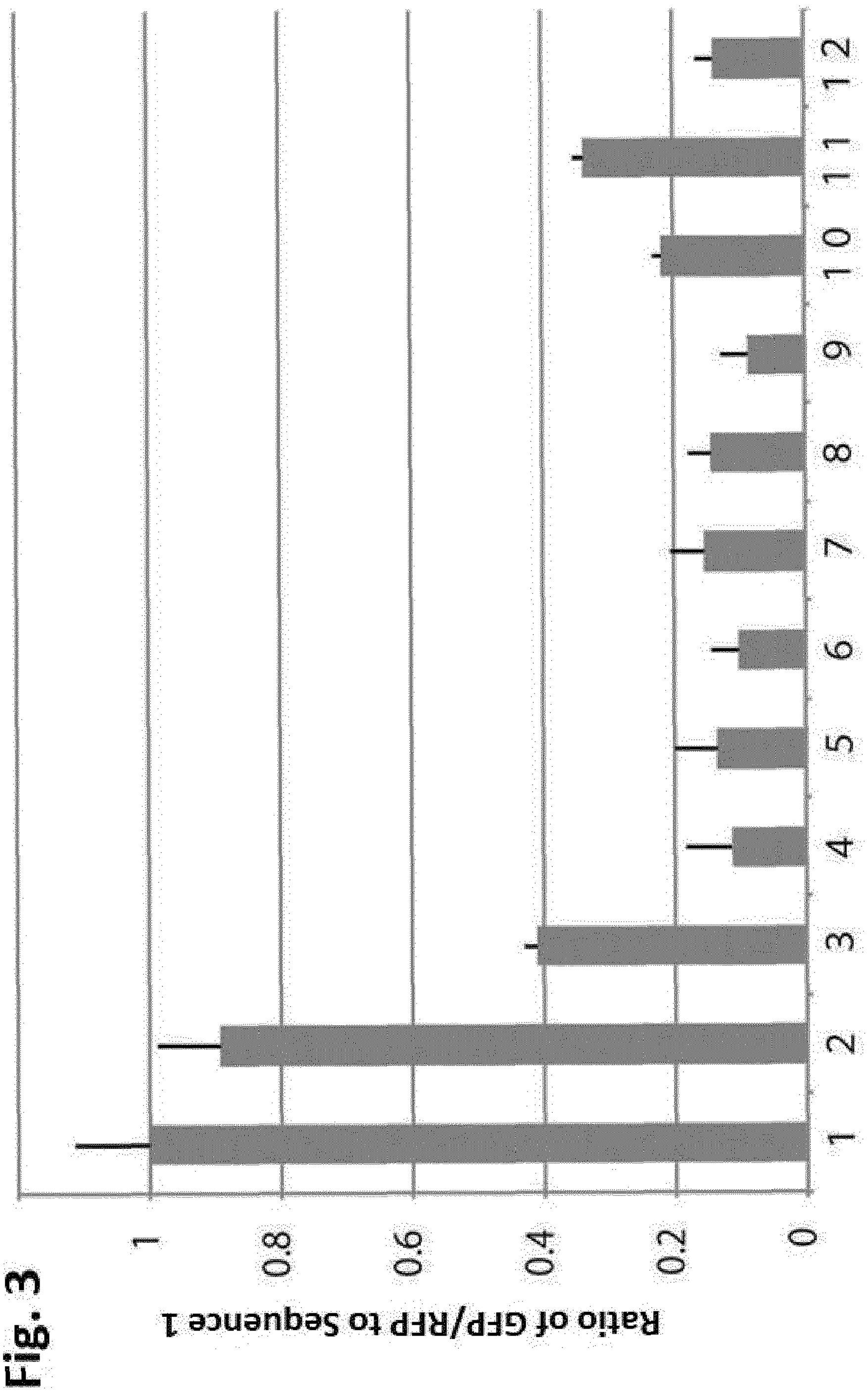

**INTERNATIONAL SEARCH REPORT**

International application No.
PCT/JP2020/009553

A. CLASSIFICATION OF SUBJECT MATTER
A01H 1/00(2006.01)i; C12N 5/10(2006.01)i; C12N 15/82(2006.01)i; C12Q 1/6869(2018.01)i
FI: C12N15/82 100Z; C12Q1/6869 Z; A01H1/00 A; C12N5/10 ZNA
According to International Patent Classification (IPC) or to both national classification and IPC

B. FIELDS SEARCHED
Minimum documentation searched (classification system followed by classification symbols)
A01H1/00; C12N5/10; C12N15/82; C12Q1/6869

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamLII); CAplus/MEDLINE/EMBASE/BIOSIS/WPIDS (STN); GenBank/EMBL/DDBJ/GeneSeq; WPIDS/WPIX (STN)

C. DOCUMENTS CONSIDERED TO BE RELEVANT

| Category* | Citation of document, with indication, where appropriate, of the relevant passages | Relevant to claim No. |
|---|---|---|
| X<br>Y | SWIATECKA-HAGENBRUCH, Monika et al, "High diversity of plastidial promoters in Arabidopsis thaliana", Mol Genet Genomics, 2007, vol. 277, pp. 725-734 page 726, left column, lines 35-49, page 730, right column, lines 18-23 | 5-10<br>1-4 |
| X<br>Y | GHULAM, Mustafa Malik et al., "Transcriptional organization of the large and the small ATP synthase operons, atpI/H/F/A and atpB/E, in Arabidopsis thaliana chloroplasts", Plant Mol.Biol., 2012, vol. 79, pp. 259-272 fig. 1-3 | 5-10<br>1-4 |
| X<br>Y | GERASYMENKO, I. M. et al., "Comparison of effectiveness of 50-regulatory sequences in transplastomic tobacco chloroplasts", Transgenic Res., 2017, vol. 26, pp. 65-75 fig. 2 | 5-10<br>1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
27 May 2020 (27.05.2020)

Date of mailing of the international search report
09 June 2020 (09.06.2020)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

INTERNATIONAL SEARCH REPORT

International application No.
PCT/JP2020/009553

C (Continuation). DOCUMENTS CONSIDERED TO BE RELEVANT

| Category* | Citation of document, with indication, where appropriate, of the relevant passages | Relevant to claim No. |
|---|---|---|
| X | TSENG, Ching-Chih et al., "Differential regulation of Arabidopsis plastid gene expression and RNA editing in non-photosynthetic tissues", Plant Mol. Biol., 2013, vol. 82, pp. 375-392 table 2 | 5-10 |
| Y | XUE, Mande et al., "Isolation and Characterization of a Green-Tissue Promoter from Common Wild Rice (Oryza rufipogon Griff.)", Int. J. Mol. Sci., 2018, vol. 19, 2009; doi:10.3390/ijms19072009, pp. 1-13 materials and Methods | 1-4 |
| Y | ZHANG, Ning et al., "Isolation and characterization of "GmScream" promoters that regulate highly expressing soybean (Glycine max Merr.) genes", Plant Science, 2015, vol. 241, pp. 189-198 methods | 1-4 |
| Y | ZHAO, Yanxin et al., "Genome-Wide Identification, Evolution and Expression Analysis of mTERF Gene Family in Maize", PLOS ONE, 2014, vol. 9, e94126, pp. 1-19 page 9, left column, lines 2-4, fig. 5 | 1-4 |

Form PCT/ISA/210 (continuation of second sheet) (January 2015)

**INTERNATIONAL SEARCH REPORT**

International application No.
PCT/JP2020/009553

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No.
PCT/JP2020/009553

<Continuation of Box No. III>

(Invention 1) Claims 1-4

Claims 1-4 have a special technical feature of a "method for selecting promoters functioning in organelles, the method including: a step of mapping sequence information onto a sequence of organelle DNA; and a step of identifying an upstream region of a selected region as a promoter functioning in organelle". Thus, claims 1-4 are classified as invention 1.

(Inventions 2-13) Parts related to sequence numbers 1-12 in claims 5-7

Parts related to sequence numbers 1-12 in claims 5-7 are not considered to share identical or corresponding special technical features with invention 1.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013077420 A1
- JP 2019042535 A


### Non-patent literature cited in the description


- **DAY, A. ; GOLDSCHMIDT-CLERMONT, M.** *Plant Biotechnol. J.,* 2011, vol. 9, 540
- **MORTAZAVI A. et al.** *Nat Methods.,* 2008, vol. 5 (7), 621-628
- **DI C. et al.** *Plant J.,* 2014, vol. 80 (5), 848-861
- **JAVIS P. ; LOPEZ-JUEZ E.** *Nat. Rev. Mol. Cell Biolo.,* 2013, vol. 14, 787-802
- **SVAB et al.** *Proc. Nal. Acad. Sci. USA,* 1990, vol. 87, 8526
- **SIKDAR et al.** *Plant Cell Rep.,* 1998, vol. 18, 20
- **SIDOROV et al.** *Plant J.,* 1999, vol. 19, 209
- **RUF, S. et al.** *Nature biotechnol.,* 2001, vol. 19, 870
- **HOU et al.** *Transgenic Res.,* 2003, vol. 12, 111
- **HAJDUKIEWICZ P. et al.** *Plant Mol Biol.,* 1994, vol. 25, 989-94
- **SVAB Z. et al.** *Proc Natl acad Sci USA,* 1990, vol. 87, 8526-8530
- **GOLDS T. et al.** *Bio Technol.,* 1992, vol. 11, 95-97
- **CHUAH J.A. et al.** *Scientific Reports,* 2015, vol. 5, 7751
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- *Proc. Natl. Acad. Sci. USA,* 1980, vol. 77, 4505
- *Proc. Natl. Acad. Sci. USA,* 1984, vol. 81, 5330
- *Nature,* 1962, vol. 195, 788
- **TOKI S. et al.** *Plant Physiol.,* 1992, vol. 100 (3), 1503-1507
- **CHRISTOU P. et al.** *Bio/Technology,* 1991, vol. 9, 957-962
- **HIEI Y. et al.** *Plant J.,* 1994, vol. 6, 271-282
- *Methods Mol Biol.,* 2014, vol. 1132, 205-220